# EUROPEAN PATENT SPECIFICATION

(11) **EP 2 605 736 B1**
(45) Date of publication and mention of the grant of the patent: **05.04.2017**
(21) Application number: 11818491.0
(22) Date of filing: 21.02.2011
(51) Int. Cl.: A61F 13/02

(54) **TEXTILE COMPOSITE MATERIAL COMPRISING NANOFIBER NONWOVEN**
TEXTILVERBUNDMATERIAL MIT EINEM NANOFASERVLIES
MATÉRIAU COMPOSITE TEXTILE COMPRENANT UN NON-TISSÉ DE NANOFIBRES

(30) Priority: 20.08.2010 WO PCT/EP2010/005135
(43) Date of publication of application: 26.06.2013
(73) Proprietor: SNS Nano Fiber Technology, LLC, Hudson OH 44236 (US)
(72) Inventor: LADEMANN, Jurgen, D-10117 Berlin (DE); FRAZIER, Laura, M., Stow OH 44224 (US); KATAPHINAN, Woraphon, Cuyahoga Falls OH 44224 (US)
(74) Representative: Prinz & Partner mbB
(86) International application number: PCT/US2011/025615
(87) International publication number: WO 2012/023996

(56) References cited:
- EP-A1- 1 953 286
- WO-A1-2011/023342
- DE-A1-102005 036 992
- DE-A1-102007 024 220
- US-A- 5 720 832
- US-A1- 2003 220 048
- US-A1- 2005 079 379
- US-A1- 2005 266 760
- US-A1- 2006 094 320
- US-A1- 2007 026 753
- US-A1- 2008 255 531
- US-A1- 2009 093 585
- US-A1- 2009 227 969

## Description

### BACKGROUND OF THE INVENTION

Previously, a decontamination of skin after contact with hazardous substances released by chemical and reactor accidents, for example, is carried out mainly by intensive washing using different detergents and solvents. However, this way of proceeding has the disadvantage that it is hardly possible or not possible at all to extract particles of noxious substances which have already penetrated the hair follicles or the upper skin cell layer, *i.e.* the upper corneocytes of the stratum corneum, which constitute a long-term reservoir for topically applied substances. Furthermore, in case of a decontamination, the noxious substances which are to be removed from the skin surface are partially rubbed in the hair follicles and the skin furrows by an intensive washing. In this way, the long-term effect of the noxious substances in the skin may even be increased. Additionally, efficient methods for skin decontamination may be not only important in the case of industrial or research accidents, but also in relation to terrorist attacks. Accordingly, there is a need to provide a material and method for decontaminating the skin which permits an effective removal of noxious substances from the skin surface without washing.

As used herein, the term "decontamination" or "decontaminating" denotes substantial removal of contaminants or noxious substances on a surface to be decontaminated or cleaned. In general, decontamination removes at least 30% (e.g., at least 50%, at least 70%, at least 80%, at least 85%, or at least 90%) of the contaminants or noxious substances.

As used herein, the term "noxious substances" or "contaminants" refers to substances that are neither a natural component nor an integral part of a surface to be cleaned or decontaminated. They can be, e.g., harmful substances such as poison or other toxins or foreign contaminants that are undesirable on the skin. They can be in a liquid form (e.g., based in water or oil), emulsion, cream, colloidal, or solid particles.

Nonwoven fabric is a fabric-like material made from long fibers, bonded together by chemical, mechanical, heat or solvent treatment. The term is used to denote fabrics, such as felt, which are neither woven nor knitted. Nonwovens made of textile fibers having a diameter of less than 10 µm, preferably of less than 1 µm, are usually referred to as "nanofiber nonwovens." Nanofiber nonwovens are described, for example, in U.S. Patent No. 4,043,331 and PCT Application Publication No. WO 01/27365. These documents also disclose methods for manufacturing of these nonwovens and are incorporated herein by reference in their entireties.

As used herein, the term "superabsorbent" denotes materials which can absorb and retain extremely large amounts of water or other fluids or liquids (e.g., up to a thousand times of their own mass). When the material is a polymer, such a superabsorbent can absorb water or another liquid and swell to form a gel. Superabsorbents (including superabsorbent polymers) and methods of their manufacture are well known in the art. See, e.g., Ullmanns Encyclopedia of Industrial Chemistry, 6th Ed., Vol. 35, pp. 73 ff., 2003; Modern Superabsorbent Polymer Technology (1 ed.), Fredric L. Buchholz and Andrew T. ; Graham (Ed.), John Wiley & Sons, 1997. Examples of superabsorbent include poly-acrylic acid sodium salt, polyacrylamide copolymers (e.g., polyacrylate/polyacrylamide copolymer), ethylene maleic anhydride copolymer, cross-linked carboxymethylcellulose, polyvinyl alcohol copolymers, cross-linked polyethylene oxide, and starch grafted copolymer of polyacrylonitrile.

DE - A 102005054698 discloses a nanofiber nonwoven finished with a superabsorbent, which can be used for absorption or slow release of different fluids, e.g., body liquids such as sweat. It describes superficially post-cured superabsorbents which have a more strongly post-cured shell and a less strongly postcured core which serves to absorb fluids. Compared to superabsorbents that are not post-cured, superabsorbents having such a structure show a smaller "gel blocking" effect. This effect is caused by a clogging of swollen superabsorbent particles or superabsorbent particles which have started to swell, and it has a negative impact on the absorbency and the retention ability of the superabsorbent.

WO 2011/023342 is a post-published document and discloses an absorbent textile composite material for decontaminating the skin, comprising a flexible carrier layer and an active layer connected with the carrier layer. The active layer comprises a nanofiber nonwoven filled with a superabsorbent to absorb and retain noxious substances.from the skin. The textile composite is used in a method for decontaminating of the skin from noxious substances without a washing and/or massage procedure.

Accordingly, the use of a nanofiber nonwoven in a textile composite material, wherein the nanofiber nonwoven is filled with a superabsorbent, would allow for the decontaminating of the skin from noxious substances without washing. As used herein, the term "skin" denotes to either the skin of a mammal (e.g., a human) or a subject's physical surface through which water or another liquid can penetrate.

### SUMMARY OF THE INVENTION

The following presents a simplified summary in order to provide a basic understanding of some aspects of the disclosed innovation. This summary is not an extensive overview, and it is not intended to identify key or critical elements or to delineate the scope thereof. Its sole purpose is to present some concepts in a simplified form as a prelude to the more detailed description that is presented later.

In one aspect, the invention provides absorbent textile composite materials, each comprising a carrier layer and an active layer, wherein the active layer is connected with the carrier layer and comprises a nanofiber nonwoven optionally filled with a superabsorbent. The carrier layer comprises cellulose, derivatives of cellulose, polyglycolide, polylactic acid, polyethylene adipate, polyhydroxyalkanoate, polybutylene terephthalate, polytrimethylene terephthalate, polyethylene naphthalate, or a mixture or copolymer thereof. Further, the carrier layer is impermeable to water, and the nanofiber nonwoven comprises fibers with a diameter of between 0.001 µm and 10 µm.

It has been found by the inventors that the optional superabsorbent was very capable of absorbing or capturing (chemically or physically) liquid or oily substance from a surface or skin to be decontaminated or cleaned, and the nanofiber nonwoven structure provides physical space or sometime electronic charge that makes it suitable for absorbing or trapping solid particles. Alternatively, the nanofiber nonwoven may absorb or trap solid particles through Van der Waals forces.

A "carrier layer" refers to a layer that is designed and positioned to carry the "active layer". It can have the same or different chemical composition as compared to an active layer, and it may or may not contain or include a superabsorbent.

In some embodiments, the carrier layer and the active layer are integrally formed with each other. In some other embodiments, the carrier layer and the active layer are bonded to each other.

In some embodiments, the carrier layer is impermeable to oil vapor. In some other embodiments, the carrier layer is permeable to heat radiation. In still some other embodiments, the carrier layer is heat-conducting. For instance, the carrier layer may comprise metal or ceramics.

In some embodiments, the active layer further includes an agent that is capable of stimulating production of sweat on a skin. In some other embodiments, the active layer further includes a metallic coating. In still some other embodiments, the carrier layer is elastic. In some other embodiments, the carrier layer is flexible. In yet still some other embodiments, the carrier layer is not expandable.

As used herein, the term "production of sweat" can be applied to not only a mammal skin that naturally can produce or excrete sweat, but also a non-mammal-skin surface through which fluid can penetrate or permeate under certain condition.

In some embodiments, the superabsorbent contained in the composite material includes polymer particles which have a core swelling in the presence of water and a superficially postcured shell. The polymer particles can be a screening fraction of polymer particles which have not been crushed after superficial postcure of the shell.

In some other embodiments, the superabsorbent can include a starch graft polymer, a biodegradable superabsorbent, activated carbon, clay, aluminum oxide, ionic exchange resin, or polyacrylate. Examples of biodegradable superabsorbent include, e.g., cross-linked copolymer of poly(carboxymethylcellulose) or poly(hydroxyethylcellulose), and carbodiimide. See, e.g., US 20080227944 which is incorporated herein by reference in its entirety.

In some embodiments, the nanofiber nonwoven contained in the composite material includes cellulose or its derivative, polyurethane (e.g., aromatic or aliphatic, polyester- or polyether-based polyurethanes), polyamide, polyester, polyacrylonitrile, polyvinyl alcohol, poly vinylpyrrolidone, poly ethyleneoxide, cellulose acetate, poly(ethylenimine), poly(caprolactone), poly(2-hydroxymethacrylate), or a mixture or copolymer thereof.

In some embodiments, the absorbent textile composite material further includes an adhesive layer. The adhesive layer can be, e.g., positioned on the carrier layer (away from the active layer) or on the active layer, and will enable the composite material to adhere to or connect with a surface, e.g., a skin area. Accordingly, in some other embodiments, the carrier layer in the composite material is larger than the active layer and is provided with an adhesive layer encircling its rim; while in some other embodiments, an adhesive layer is provided and positioned on the active layer.

In some other embodiments, the active layer further includes an agent (or a color indicator) to indicate the presence of water or other liquid in the active layer. When such a composite is applied on a skin area, the color change of the agent (or color indicator) could indicate the production of sweat.

In some embodiments, the absorbent textile composite material further includes at least one layer of a nanofiber nonwoven without the superabsorbent. This additional layer of a nanofiber nonwoven can be configured or positioned, e.g., to attach or connect to the active layer (which may have superabsorbent) on the opposite of the carrier layer.

In some embodiments, the nanofiber nonwoven comprises fibers with a diameter of between about 0.001 µm and about 10 µm (e.g., between about 0.1 µm and about 1.5 µm, or between about 300 nm and about 900 nm.

In some embodiments, the nanofiber nonwoven has an average pore size of between about 0.01 µm and about 500 µm (e.g., less than about 250 µm, or less than 100 µm).

In some embodiments, the nanofiber nonwoven has a porosity of between about 10% and about 90% (e.g., between about 40% and about 90%, or between about 70% and about 90%).

In some embodiments, the nanofiber nonwoven has a density of between about 0.8 g/cm³ and about 1.5 g/cm³.

In some embodiments, the nanofiber nonwoven has a mass per unit area of between about 5 g/m² and about 1,000 g/m² (e.g., between about 50 g/m² and about 500 g/m², between about 50 g/m² and about 400 g/m²; or between about 150 g/m² and about 250 g/m²).

In some embodiments, the nanofiber nonwoven has a breaking force and elongation of 0.1 to 100.0 MPa and 100 to 2,000% (e.g., 0.5 to 5.0 MPa and 250 to 1,000%, or 1.5 to 2.0 MPa and 400 to 500%), as measured in a strip method according to EDANA standard WSP 110.4 (05).

In some embodiments, the nanofiber nonwoven has an absorbency in saline of between about 0.1 g/g and about 200.0 g/g (e.g., between about 2.5 g/g and about 150.0 g/g, or between about 8.0 g/g and about 10.0 g/g), as measured in a teabag test in a 0.9% NaCl in water for 30 minutes in accordance with EDANA standard test WSP 240.2 (05).

In some embodiments, the nanofiber nonwoven has a retention capacity for saline of between about 0.1 g/g and about 100.0 g/g (e.g., between about 3.0 g/g and about 25.0 g/g, or between about 6.0 g/g and about 8.0 g/g), as measured in accordance with EDANA standard test WSP 241.2 (05).

In some embodiments, the active layer has a filling level of superabsorbent of between about 10% and about 80% (e.g., between about 40% and about 80%, or between about 50% and about 75%).

In some embodiments, the active layer has an absorbency in saline of between about 10 g/g and about 100 g/g (e.g., between about 25 g/g and about 31 g/g) at a superabsorbent filling level of 50%, or between about 20 g/g and 75 g/g (e.g., between about 38 g/g and about 45 g/g) at a superabsorbent filling level of 75%, or at least 0.01 g/g without superabsorbent.

In some embodiments, the active layer has a retention capacity for saline of between 14 g/g and 40 g/g (e.g., between 20 g/g and 35 g/g) at a superabsorbent filling level of between 50% and 75%.

In another aspect, there is disclosed a method for decontaminating a skin area contaminated with noxious substances, which includes the steps of:
applying absorbent textile composite material to the contaminated skin area for a predetermined period of time, and
removing the absorbent textile composite material from the contaminated skin area,
wherein the absorbent textile composite material comprise a carrier layer and an active layer, the active layer is connected with the carrier layer and comprises a nanofiber nonwoven filled with a superabsorbent, and the method is free of a washing procedure or a massage procedure.

In some embodiments, the absorbent textile composite material further includes an adhesive layer on the active layer facing the contaminated skin area.

In some disclosed embodiments, the method further includes applying a second absorbent textile composite material to the contaminated skin area; wherein the second absorbent textile composite material comprises a carrier layer, an active layer, and an adhesive layer; the active layer is connected with the carrier layer and comprises a nanofiber nonwoven filled with a superabsorbent; and the adhesive layer is on the active layer facing the contaminated skin area and adheres the absorbent composite material to the contaminated skin area.

In some disclosed embodiments, the method further includes stimulating production of sweat in the contaminated skin area, wherein the carrier layer in the composite material is impermeable to water vapor.

In some disclosed embodiments, the method further includes stimulating production of sweat in the contaminated skin area, wherein at least one of the active layer and the carrier layer is permeable to heat radiation.

In some disclosed embodiments, the method further includes stimulating production of sweat in the contaminated skin area, wherein at least one of an active layer and a carrier layer is heat conducting.

In some disclosed embodiments, the method further includes stimulating production of sweat in the contaminated skin area, wherein an active layer comprises a sweat promoting agent.

Still another disclosed method for decontaminating human skin from noxious substances includes the steps of:
applying a textile composite to a contaminated skin area for a predetermined period of time; and
removing the textile composite from the contaminated skin area;
wherein the textile composite comprises a nanofiber nonwoven and a superabsorbent integrated into the nanofiber nonwoven, the nanofiber nonwoven having a fiber diameter of less than 1 µm.

In some disclosed embodiments, the contaminated skin area comprises contaminated hair follicles before the textile composite is applied, and the noxious substances are removed from the hair follicles when or after the textile composite is removed.

The subject matter disclosed herein, in another aspect, comprises the use of a nanofiber nonwoven in a textile composite material for decontaminating skin contaminated with noxious substances without a washing procedure, wherein the nanofiber nonwoven is filled with a superabsorbent which is capable of absorbing and retaining the noxious substances from the skin.

In some embodiments, the textile composite material comprises an active layer comprised of the nanofiber nonwoven and the superabsorbent. Optionally, the active layer can further include at least one cover layer which is formed from a nanofiber nonwoven without a superabsorbent, and one base layer comprising the nanofiber nonwoven filled with the superabsorbent.

In some other embodiments, the active layer has a sandwich structure comprising a top and bottom cover layer formed from a nanofiber nonwoven without a superabsorbent, and a base layer(i.e., middle layer) comprising the nanofiber nonwoven filled with the superabsorbent, the base layer being arranged between said top and bottom cover layer. The cover layers can help to prevent the superabsorbent from bleeding out of the base layer, and provide a softer feel to the textile composite material.

In order to form the active layer, the superabsorbent may be dusted on the nanofiber nonwoven and mechanically held in the nanofiber structure. Alternatively, the superabsorbent can be provided or included to the nanofibers during the spinning process, or can be added to the polymer solution before spinning.

In a further aspect of the invention, the the textile composite material is used for the manufacture of a kit for decontaminating a skin area contaminated with noxious substances, in accordance with claim 22.

In some embodiments, a textile composite material used for decontaminating of skin includes a flexible carrier layer and an active layer connected to the carrier layer, and the active layer includes a nanofiber nonwoven filled with a superabsorbent that is capable of absorbing and retaining noxious substances from the skin.

Within the context of decontamination of the skin, the textile composite material is applied to a skin area contaminated by particles of noxious substances such that the active layer comes in contact with the concerned parts of the skin. The carrier layer may be used for the shaping and, owing to its flexibility, for the optimum shape adaptation of the composite material to the skin surface.

The nanofiber nonwoven contained in the active layer and finished with a superabsorbent is particularly absorbent due to the high capillarity in the nonwoven, and absorbs the noxious substances to be removed, according to the existing concentration gradient between the skin surface and the nonwoven material. The noxious substances can then be effectively stored and retained by the superabsorbent. After an appropriate decontamination time of, e.g., about 30 seconds to 30 minutes or about 1 to 5 minutes, depending on the nature of the noxious substances, the textile composite material including the absorbed noxious substances can be removed again from the skin.

Since this type of decontamination of the skin is primarily based on the absorbing effect of the nanofiber nonwoven finished with a superabsorbent, a rubbing of the noxious substances into the hair follicles and the skin furrows is avoided, and the noxious substance particles are thus prevented from further entering the hair follicles or the upper cell layer of the corneocytes. The risk of a long-term effect of the noxious substances in the skin is therefore reliably excluded.

In some embodiments of the invention, at least one of the carrier layer and the active layer are designed to stimulate the production of sweat by the skin. An increased production of sweat by the areas of the skin covered by the active layer or carrier layer increases the decontaminating effect of the textile composite material. The sweat flushes out the noxious substances having already penetrated the hair follicles and the upper cell layer of the corneocytes. The sweat, along with these noxious substances and the particles of noxious substances still present on the skin surface, is then absorbed by the superabsorbent in the nanofiber nonwoven and retained therein. As such, this could provide effective decontamination of a skin area without resorting to washing with water.

The textile composite material has a carrier layer that is impermeable to water vapor. This carrier layer can close the active layer lying on the contaminated skin surface in a damp-tight manner and thus stimulates the production of sweat in the enclosed skin area. The skin surface cannot release the sweat produced there to the environment by evaporation. Rather, the sweat, along with the noxious substances flushed out, is absorbed by the nanofiber nonwoven and retained in the superabsorbent.

According to a further embodiment, at least one of the active layer and the carrier layer are configured to be permeable to heat radiation. The skin areas concerned which are covered by the textile composite material can then be purposefully heated, e.g., using heat radiators or a chemical reaction producing heat. A quick and controlled stimulation of the production of sweat in the contaminated skin area is thereby obtained. The heat transfer between the active layer and the skin surface may take place by heat conduction. It is preferable that both the carrier layer and the active layer are permeable to heat radiation.

In some other embodiments, the carrier layer or the active layer of the textile composite material is configured so as to be heat conducting. It is, for example, possible to insert heat conducting fibers or heat conducting filaments into the carrier layer or the active layer, or provide the active layer with a heat-conducting coating. Examples of suitable heat-conducting fibers or filaments include metallic or ceramic materials. As in the embodiment described above, the skin areas concerned can be purposefully heated using an appropriate heating source such as, *e.g*., an electrical heating element, and the production of sweat can therefore be stimulated.

In still some other embodiments, the active layer may contain an agent promoting the production of sweat, e.g., by applying a sweat promoting agent onto the surface of the active layer that faces the skin. The textile composite material then acts like a transdermal system, the agent being absorbed by the skin first and stimulating the production of sweat. In contrast to transdermal systems, the sweat promoting agent does not require a long-term effect since the production of sweat is to be locally restricted and is intended to occur only for the duration of the decontamination. The sweat produced in the contaminated skin area, along with the flushed-out noxious substances and the excessive agent, is then absorbed by the nanofiber nonwoven and retained in the superabsorbent.

The embodiments described above can be combined with each other in any way. For instance, the carrier layer can be at the same time impermeable to water vapor and heat conducting or permeable to heat radiation, or the active layer can contain an agent promoting the production of sweat. Further combinations are also conceivable and considered to be within the scope of the invention.

The carrier layer and the active layer can be formed integrally with each other. For example, the carrier layer can be a woven fabric, and the nanofibers of the active layer can be firmly spun onto and with the woven fabric filaments of the carrier layer. The carrier layer and the active layer can be bonded to each other. The layers can be produced separately, so as to have the respective desired properties, and then bonded to each other by chemical, thermal or physical bonding as is generally known in the art.

In some embodiments, the carrier layer is elastic, allowing for optimum adaptation of the textile composite material to the skin. If the carrier layer is elastic, the textile composite material can be adapted to the shape of the skin surface by contraction or expansion.

In some other embodiments, the carrier layer can be configured so as not to be expandable. During the absorption of noxious substances flushed out by the sweat, the superabsorbent contained in the active layer swells as a result of which the volume of the active layer increases. Since the carrier layer which is arranged on the side of the active layer that faces away from the skin, cannot expand, this increase in volume results in that the contact between the active layer and the skin surface is intensified and the active layer rests more firmly on the skin area concerned, and thus further increases the effectiveness of the decontamination.

In some further embodiments, an adhesive layer is provided on the surface of the composite material that faces the skin to fasten the textile composite material to the skin. In this embodiment, the carrier layer preferably has a planar surface area which is larger than a planar surface area of the active layer so that the carrier layer overlaps the active layer thereby encircling the rim of the active layer. The overlapping edge part of the carrier layer is provided with the adhesive layer for connecting the composite material with the skin.

Additionally, the surface of the active layer that faces away from the carrier layer, *i.e.* the skin-side surface, can be provided with an adhesive layer. This leads to an even better contact of the active layer with the skin which in turn leads to an improvement of the decontaminating effect.

Furthermore, the adhesive layer applied to the active layer can be designed to remove the upper cell layer of the corneocytes from the skin. Accordingly, in some embodiments, the composite material also includes an adhesive layer on the active layer that can be used to remove the upper cell layer of the corneocytes from the skin to be decontaminated. When removing the textile composite system from the skin area to be decontaminated, the upper cell layer of the corneocytes, along with the noxious substance particles having already entered the latter, is thus extracted in a non-invasive way, and the proportion of the removed noxious substances is thus again increased.

In some other embodiments, the composite textile material comprises a flexible carrier layer, a first active layer, and a second active layer, wherein the first active layer is provided with a metal coating. The nanofiber nonwoven included in or constituting the first active layer is produced to form peaks and valleys. The valleys can be filled with the second active layer. Although at least the second active layer is composed of the nanofiber nonwoven filled with superabsorbent, both the first and second active layers can include the superabsorbent. The adhesive layer is provided on the metallic coating, or on the rim portion of the carrier layer, as described above.

The active layer may also comprise a color indicator to indicate the production of sweat. Owing to this addition, the optimum time to remove the material from the skin can be indicated to the user of the textile composite material. The colors of the color indicator change if the nanofiber nonwoven has absorbed an amount of sweat that is sufficient for decontaminating or the capacity of the superabsorbent to absorb liquid has been exhausted. The color indicator may be combined with the superabsorbent and incorporated into the active layer, e.g., by adding the combined superabsorbent and color indicator to the polymer melt or solution before spinning of the nanofiber nonwoven.

The textile composite material can be configured as or made in the form of a cloth, a compress, a dressing, a plaster, or an article of clothing or part of clothing for application on larger surfaces.

In some embodiments, the superabsorbent polymer particles are a screening fraction having a particle size distribution of d50 = 55-100 µm and d100 = 100-150 µm, and are not crushed after the superficial postcure of the shell, prior to their incorporation into the nanofiber nonwoven.

The nanofiber nonwovens can be produced through melt spinning, electrospinning, or gas jet spinning (NGJ) of suitable polymers. It is also contemplated that part of the nanofibers in the nonwoven can be replaced by microfibers which have a larger diameter than nanofibers.

The superabsorbent may be dusted onto a sheet of the nanofiber nonwoven and mechanically integrated into the nanofiber structure. This process can be repeated until the desired filling level of superabsorbent is achieved. Alternatively, the superabsorbent can be applied to the nanofibers during the spinning process when the fiber dries and solidifies. Or, the superabsorbent can be homogeneously dispersed in a polymer solution which is then subjected to spinning into a nanofiber nonwoven including the superabsorbent embedded in the fiber structure.

In some embodiments of the composite material that can be used for decontaminating the skin, the nanofiber nonwoven contained therein has at least one or more of the following physical properties: a fiber diameter of between 0.001 µm and 10 µm (e.g., between 0.1 µm and 1.5 µm, or between 300 nm and 900 nm; an average pore size of between 0.01 µm to 500 µm (e.g., preferably less than 250 µm, more preferably less than 100 µm; a porosity (i.e., , the percentage of the total volume of the nonwoven which is free space) of between 40% and 90% (e.g., between 70% and 90%); a thickness of the active layer of between 0.1 mm to 2 mm; a density of between 0.8 to 1.5 g/cm³; a mass per unit area of between 50 to 500 g/m² (e.g., between 50 and 400 g/m², or between 150 to 250 g/m²), a breaking force and elongation (strip method), according to EDANA standard WSP 110.4 (05), of 1.5 - 2 MPa and 485 to 500%; an absorbency (tb) in saline (0.9% NaCl in water, 30 min), as measured in the teabag test in accordance with EDANA standard test WSP 240.2 (05), of between about 8 g/g and 10 g/g; a retention capacity (CRC),for saline (0.9% NaCl in water, 30 min), as measured in accordance with EDANA standard test WSP 241.2 (05), of between about 6 g/g to 8 g/g.

In some other embodiments, the active layer composed of the nanofiber nonwoven filled with the superabsorbent preferably has at least one or more of the following properties: a filling level of superabsorbent (SAP), that is calculated as weight by weight of dry material, of between about 10% and 80% (e.g., between about 40% and 80%, or between about 50% to 70%); an absorbency (tb) in saline (0.9% NaCl in water, 30 min), as measured in the teabag test in accordance with EDANA standard test WSP 240.2 (05), of between 20 g/g and 50 g/g (e.g., between 25 g/g and 31 g/g) at an SAP filling level of 50%, or between about 38 g/g and 45 g/g at an SAP filling level of 75%; a retention capacity (CRC) for saline (0.9% NaCl in water, 30 min), as measured in the centrifuge test according to EDANA standard test WSP 241.2 (05), of between 14 g/g and 40 g/g (e.g., between 20 g/g and 35 g/g) for SAP filling levels of between 50% and 75%; and a contact angle of between 110° and 125°, as measured at 22 °C and 55% relative humidity (Fibro DAT™ of Rycobel, Belgium).

As used herein, the term "or" is meant to include the meaning of "and."

As used herein, a singular term is meant to include the meaning of its plural form.

To the accomplishment of the foregoing and related ends, certain illustrative aspects of the disclosed innovation are described herein in connection with the following description and the annexed drawings. These aspects are indicative, however, of but a few of the various ways in which the principles disclosed herein can be employed and is intended to include all such aspects and their equivalents. Other advantages and novel features will become apparent from the following detailed description when considered in conjunction with the drawings.

### BRIEF DESCRIPTION OF THE DRAWINGS

Figure 1 illustrates a schematic cross-sectional view of the textile composite material in accordance with principles of the present invention;
Figure 2 illustrates a schematic cross-sectional view of another embodiment of the textile composite material in accordance with principles of the present invention, and
Figure 3 illustrates a schematic view of the bottom surface of a further embodiment of the textile composite material in accordance with principles of the present invention.
Figure 4 illustrates a schematic cross sectional view of a further embodiment of the textile composite material in accordance with principles of the present invention.
Figure 5 illustrates a bottom view of the embodiment of Fig. 4 in accordance with principles of the present invention.
Figure 6 is a graph illustrating the penetration profile of a model substance into the skin obtained by tape stripping.
Figure 7 is a graph illustrating the distribution of the model substance in the stratum corneum after washing.
Figure 8 is a graph illustrating the distribution of the model substance in the stratum corneum after decontamination with a textile composite material composed of nanofiber nonwoven filled with superabsorbent.
Figure 9 illustrates a laser scanning microscope (LSM) image of the distribution of a fluorescent dye on the skin after application and penetration.
Figures 10a-b illustrate an LSM image of the distribution of the fluorescent dye after washing.
Figures 11a-c illustrate an LSM image of the distribution of the fluorescent dye after decontamination with the textile composite material.
Figure 12 is a flow chart illustrating the method of decontaminating human skin from noxious substances.
Figure 13 is a flow chart illustrating further steps in the method of decontaminating human skin from noxious substances.

### DETAILED DESCRIPTION OF THE INVENTION

The innovation is now described with reference to the drawings, wherein like reference numerals are used to refer to like elements throughout. In the following description, for purposes of explanation, numerous specific details are set forth in order to provide a thorough understanding thereof. It may be evident, however, that the innovation can be practiced without these specific details. In other instances, well-known structures and devices are shown in block diagram form in order to facilitate a description thereof.

Typically, a decontamination of the skin after contact with noxious substances by intensive washing using different detergents and solvents, does not extract particles of noxious substances which have already penetrated the hair follicles or the upper skin cell layer, *i.e.* the upper corneocytes of the stratum corneum, which constitute a long-term reservoir for topically applied substances. Usually, hair follicles act as a long-term reservoir for topically applied substances providing significantly increased storage times in comparison to the stratum corneum. Additionally, the hair follicles contain or are surrounded by several important target structures, such as blood capillaries, stem and dendritic cells. Here, hazardous substances can exhibit strong destructive activities. Furthermore, in case of a decontamination, the noxious substances which are to be removed from the skin surface are partially rubbed in the hair follicles and the skin furrows by an intensive washing. In this way, the long-term effect of the noxious substances in the skin may even increase. Thus, a material for decontaminating the skin which permits an effective removal of noxious substances from the skin surface must not utilize washing.

Accordingly, the absorbent textile composite material disclosed does not utilize washing to decontaminate the skin. The textile composite material comprises a carrier layer and an active layer connected to the carrier layer. The active layer comprises a nanofiber nonwoven filled with a superabsorbent capable of absorbing and retaining noxious substances from the skin. One advantage of decontamination with the absorbent textile composite materials is that it can be carried out immediately without the utilization of water, which is not always available at the required time. Efficient methods for skin decontamination may be not only important in the case of industrial or research accidents, but also in relation to terrorist attacks.

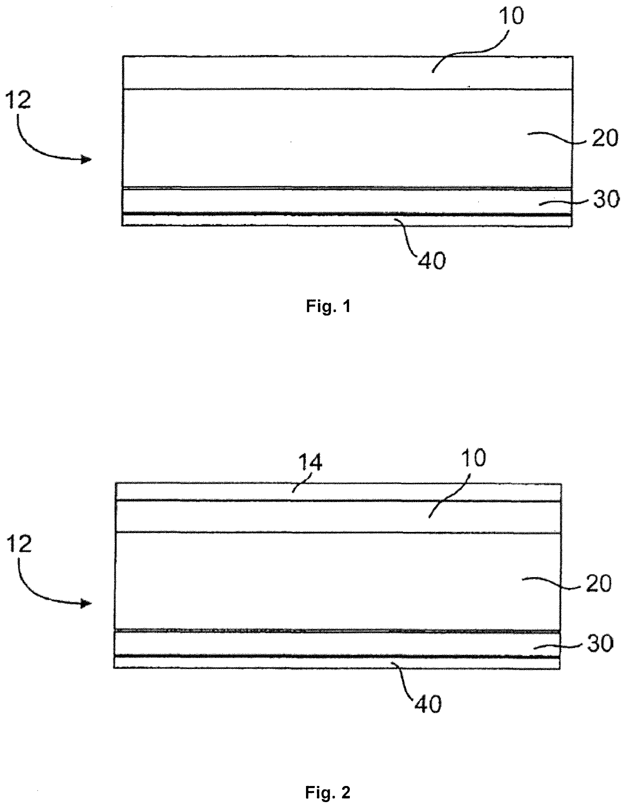

Referring initially to the drawings, Figure. 1 illustrates an absorbent textile composite material **12.** The absorbent textile composite material **12** includes a carrier layer **10** and an active layer **20** and, optionally, an adhesive layer **30** and a protective layer **40.** The carrier layer **10** may be a film or a textile web. Specifically, the carrier layer **10** is hydrophobic and made of a material impermeable to water vapor. The carrier layer **10** which is impermeable to water vapor closes the active layer **20** lying on the contaminated skin surface in a damp-tight manner and thus stimulates the production of sweat in the enclosed skin area. The skin surface cannot release the sweat produced there to the environment by evaporation. Rather, the sweat, along with the flushed-out noxious substances, is absorbed by a nanofiber nonwoven and retained in a superabsorbent. Alternatively, the carrier layer **10** may include a coating or an intermediate layer impermeable to water vapor.

In addition, at least one of the carrier layer **10** or the active layer **20** can be configured to be permeable to heat radiation or heat conducting or both. The skin areas concerned which are covered by the textile composite material **12** can then be purposefully heated, for example, using heat radiators or a chemical reaction producing heat. A quick and controlled stimulation of the production of sweat in the contaminated skin area is thereby obtained. The heat transfer between the carrier layer **10** and the skin surface may take place by heat conduction. It is preferable that both the carrier layer **10** and the active layer **20** are permeable to heat radiation.

Further, at least one of the carrier layer **10** and the active layer **20** of the textile composite material **12** can be configured to be heat conducting. It is, for example, possible to insert heat conducting fibers or heat conducting filaments into the carrier layer **10** or the active layer **20,** or provide the active layer **20** with a heat conducting coating. As in the embodiment described above, the skin areas concerned can be purposefully heated using appropriate heating sources such as, *e.g*., an electrical heating element, and the production of sweat can therefore be stimulated. The active layer **20** may also comprise a color indicator to indicate the production of sweat. Owing to this addition, the optimum time to remove the material from the skin can be indicated to the user of the textile composite material **12.** Examples of color indicators appropriate for this use include: cobalt (II) chloride, quinizarin powder, pentamethoxy red, methyl yellow, phenolphthalein, thymolphthalein, p-naphtolbenzein, 4-nitrophenol, 3-nitrophenol, o-cresolphthalein, m-cresol red, thymol blue, m-cresol purple, or mixtures thereof, the colors of which change if the nanofiber nonwoven has absorbed an amount of sweat that is sufficient for decontaminating or the capacity of the superabsorbent to absorb liquid has been exhausted. The color indicator may be combined with the superabsorbent and incorporated into the active layer **20,** e.g., by adding the combined superabsorbent and color indicator to the polymer melt or solution before spinning of the nanofiber nonwoven.

The embodiments described above can be combined with each other in any way. For instance, the carrier layer **10** can be at the same time impermeable to water vapor and heat conducting or permeable to heat radiation, or the active layer **20** can contain an agent promoting the production of sweat. Further combinations are also conceivable and considered to be within the scope of the invention.

The carrier layer **10** and the active layer **20** can be formed integrally with each other. For example, the carrier layer **10** can be a woven fabric, and the nanofibers of the active layer 20 can be firmly spun onto and with the woven fabric filaments of the carrier layer **10.** Preferably, the carrier layer **10** and the active layer **20** are bonded to each other. The layers can then be produced separately, so as to have the respective desired properties, and then bonded to each other by chemical, thermal or physical bonding as is generally known in the art.

Further, in a preferred embodiment, the carrier layer **10** is elastic, allowing for optimum adaptation of the textile composite material **12** to the skin. If the carrier layer **10** is elastic, then the textile composite material 12 can be adapted to the shape of the skin surface by contraction or expansion.

In another embodiment, the carrier layer **10** is configured to not be expandable. During the absorption of noxious substances flushed out by the sweat, the superabsorbent contained in the active layer **20** swells as a result of which the volume of the active layer **20** increases. Since the carrier layer **10** which is arranged on the side of the active layer **20** that faces away from the skin, cannot expand, this increase in volume results in that the contact between the active layer **20** and the skin surface is intensified and the active layer **20** rests more firmly on the skin area concerned, and thus further increases the effectiveness of the decontamination.

Additionally, the active layer **20** of the absorbent textile composite material **12** comprises a nanofiber nonwoven finished with a superabsorbent. The superabsorbent preferably comprises completely or mainly of polymer particles which have a skin-core structure, that is a core swelling in the presence of water and a superficially post-cured shell, and thus has a low "gel blocking" effect. The polymer particles are preferably a screening fraction of such polymer particles that have not been crushed after the superficial post-cure of the shell, prior to incorporation in the active layer 20. The polymer particles preferably have a particle size distribution of d50 = 55-100 µm and d100 = 100-150 µm and comprise (meth)acrylate or (meth)acrylate copolymer, in particular sodium polyacrylate. That is, preferably 50% of the particles have a particle size of maximum 55-100 µm, and 100% of the particles have a particle size of maximum 100-150 µm. Further, the superabsorbent may be selected from starch graft polymers such as Waterlock™, biodegradable superabsorbents, activated carbon, clay, aluminum oxide, ionic exchange resins or polyacrylates. Examples of biodegradable superabsorbent include, e.g., cross-linked copolymer of poly(carboxymethylcellulose) or poly(hydroxyethylcellulose), and carbodiimide. The nanofiber nonwoven of the active layer 20 finished with the superabsorbent is made of superfine fibers or superfine filaments having a diameter of less than 10 µm, preferably of less than 1 µm, more preferably of between about 300 nm and about 900 rim and is most preferably comprised of electrostatically spun nanofibers. The nanofiber nonwoven preferably comprises fibers or filaments formed from a thermoplastic, hydrophilic or hydrophilized polymer. Most preferably, the nanofiber nonwoven is formed from polyurethane.

The nanofiber nonwovens can be produced through melt spinning, electrospinning, or gas jet spinning (NGJ) of suitable polymers. It is also contemplated that part of the nanofibers in the nonwoven can be replaced by microfibers. Materials for producing the nanofiber nonwovens comprise thermoplastic polymers that can be polyurethane, polyamides, polyesters, polyacrylonitrile, polyvinyl alcohol, poly vinylpyrrolidone, poly ethyleneoxide, cellulose acetate, poly(ethylenimine), poly(caprolactone), poly(2-hydroxymethacrylate), or a mixture or copolymer thereof. Polyurethane is particularly preferred.

In order to form the active layer **20,** the superabsorbent may be dusted onto a sheet of the electrostatically spun nanofiber nonwoven and mechanically integrated into the nanofiber structure. Most preferably, the superabsorbent is homogeneously dispersed in a polymer solution which is then subjected to electrostatically spinning into a nanofiber nonwoven including the superabsorbent embedded in the fiber structure.

The nanofiber nonwoven contained in the active layer **20** and finished with a superabsorbent is particularly absorbent due to the high capillarity in the nonwoven, and absorbs the noxious substances that have to be removed, according to the existing concentration gradient between the skin surface and the nonwoven material. The noxious substances can then be effectively stored and retained by the superabsorbent. After an appropriate decontamination time, e.g., of about 30 seconds to 30 minutes (e.g., about 1 to 5 minutes), depending on the nature of the noxious substance, the textile composite material **12** including the absorbed noxious substances can be removed again from the skin.

Since this type of decontamination of the skin is based on the absorbing effect of the nanofiber nonwoven finished with a superabsorbent, a rubbing of the noxious substances into the hair follicles and the skin furrows is avoided, and the noxious substance particles are thus prevented from further entering the hair follicles or the upper cell layer of the corneocytes. The risk of a long-term effect of the noxious substances in the skin is therefore reliably excluded.

Furthermore, the adhesive layer **30** is provided on the surface of the composite material **12** that faces the skin to connect the composite material **12** with the skin and preferably comprises a skin-friendly adhesive, particularly preferably an acrylate adhesive. The addition of the adhesive layer **30** leads to an even better contact of the active layer **20** with the skin which in turn leads to an improvement of the decontaminating effect.

The protective layer **40** is optional and is removed before applying the textile composite system **12** to the skin. Furthermore, the textile composite material **12** can be configured as a cloth, a compress, a dressing or a plaster, also as an article of clothing or part of clothing for application on larger surfaces.

In view of the intended use of the textile composite material for decontaminating the skin, the nanofiber nonwoven preferably has at least one or more of the following physical properties: a fiber diameter of between 0.001 µm and 10 µm (e.g., between 0.1 µm and 1.5 µm or between 300 nm and 900 nm); an average pore size of between 0.01 µm to 500 µm (e.g., less than 250 µm or less than 100 µm); a porosity (i.e., a percentage of the total volume of the nonwoven which is free space) of between 40% and 90% (e.g., between 70% and 90%); a thickness of the active layer of between 0.1 mm to 2 mm; a density of between 0.8 to 1.5 g/cm³; a mass per unit area of between 50 to 500 g/m² (e.g., between 50 and 400 g/m² or between 150 to 250 g/m²); a breaking force and elongation (strip method), according to EDANA standard WSP 110.4 (05), of 1.5 - 2 MPa and 485 to 500%; an absorbency (tb) in saline (0.9% NaCl in water, 30 min), as measured in the teabagtest in accordance with EDANA standard test WSP 240.2 (05), of between about 8 g/g and 10 g/g; and a retention capacity (CRC) for saline (0.9% NaCl in water, 30 min), as measured in accordance with EDANA standard test WSP 241.2 (05), of between about 6 g/g to 8 g/g.

Further, the active layer composed of the nanofiber nonwoven filled with the superabsorbent preferably has at least one or more of the following properties: a filling level of superabsorbent (SAP), that is calculated as weight by weight of dry material, of between about 10 and 80% (e.g., between about 40 and 80% or between about 50 to 70%); an absorbency (tb) in saline (0.9% NaCl in water, 30 min), as measured in the teabag test in accordance with EDANA standard test WSP 240.2 (05) of between 20 g/g and 50 g/g (e.g., between 25 g/g and 31 g/g), at an SAP filling level of 50%, or between about 38 g/g and 45 g/g at an SAP filling level of 75%; a retention capacity (CRC) for saline (0.9% NaCl in water, 30 min), as measured in the centrifuge test according to EDANA standard test WSP 241.2 (05), of between 14 g/g and 40 g/g (e.g., between 20 g/g and 35 g/g) for SAP filling levels of between 50% and 75%; and a contact angle as measured at 22 °C and 55% relative humidity (Fibro DAT™ of Rycobel, Belgium) of between 110° and 125°.

According to the embodiment shown in Figure 2, a barrier layer **14** impermeable to water vapor is provided in addition to the layers already illustrated in Figure 1. The barrier layer **14** is intended to occlude the contaminated skin area and stimulate the production of sweat. In the embodiment of Fig. 2, the barrier layer **14** is formed from a material impermeable to water vapor (e.g., polypropylene or PET). For an additional stimulation of the production of sweat, the barrier layer **14** and the carrier layer **10** can be configured so as to be permeable to heat radiation or to be heat conducting. An increased production of sweat by the parts of the skin covered by the barrier layer **14** or carrier layer **10** increases the decontaminating effect of the textile composite material **12.** The sweat flushes out the noxious substances having already penetrated the hair follicles and the upper cell layer of the corneocytes. The sweat, along with these noxious substances and the particles of noxious substances still present on the skin surface, is then absorbed by the superabsorbent in the nanofiber nonwoven and retained therein.

At least one of the barrier layer **14** and the carrier layer **10** is configured to be permeable to heat radiation. The skin areas concerned which are covered by the textile composite material **12** can then be purposefully heated using heat radiators, for example, or a chemical reaction producing heat. A quick and controlled stimulation of the production of sweat in the contaminated skin area is thereby obtained. The heat transfer between the barrier layer **14** and the skin surface may take place by heat conduction. It is preferable that both the barrier layer **14** and the carrier layer **10** are permeable to heat radiation.

Further, the carrier layer **10** or the barrier layer **14** of the textile composite material **12** can be configured to be heat conducting. It is, for example, possible to insert heat conducting fibers or heat conducting filaments into the carrier layer **10** or the barrier layer **14,** or provide the barrier layer **14** with a heat conducting coating. As in the embodiment described above, the skin areas concerned can be purposefully heated using appropriate heating sources such as, *e.g.,* an electrical heating element, and the production of sweat can therefore be stimulated.

In still another embodiment, the barrier layer **14** may contain an agent promoting the production of sweat, for example, by applying a sweat promoting agent onto the surface of the barrier layer **14** that faces the skin. The textile composite material **12** then acts like a transdermal system, the agent being absorbed by the skin first and stimulating the production of sweat. In contrast to transdermal systems, the sweat promoting agent does not require a long-term effect since the production of sweat is locally restricted and is intended to occur only for the duration of the decontamination. The sweat produced in the contaminated skin area, along with the noxious substances, is flushed out and the excessive agent, is then absorbed by the nanofiber nonwoven and retained in the superabsorbent.

Examples of suitable sweat producing agents include, but are not limited to, methyl nicotinate, 2-hydroxyethyl salicylate, methyl salicylate, ethyl salicylate, menthol B.P. or agents containing benzene derivatives as disclosed for example in JP-A 10114649.

The active layer **20** may also comprise a color indicator to indicate the production of sweat. Owing to this addition, the optimum time to remove the material from the skin can be indicated to the user of the textile composite material **12.** Examples of color indicators appropriate for this use include, but are not limited to, cobalt (II) chloride, quinizarin powder, pentamethoxy red, methyl yellow, phenolphthalein, thymolphthalein, p-naphtolbenzein, 4-nitrophenol, 3-nitrophenol, o-cresolphthalein, m-cresol red, thymol blue, m-cresol purple, or mixtures thereof. The color of the color indicator changes if the nanofiber nonwoven has absorbed an amount of sweat that is sufficient for decontaminating and/or the capacity of the superabsorbent to absorb liquid has been exhausted. The color indicator may be combined with the superabsorbent and incorporated into the active layer **20,** for example, by adding the combined superabsorbent and color indicator to the polymer melt or solution before spinning of the nanofiber nonwoven.

The embodiments described above can be combined with each other in any way. The carrier layer **10** can be at the same time impermeable to water vapor and heat conducting or permeable to heat radiation, or the barrier layer **14** can contain an agent promoting the production of sweat. Further combinations are also conceivable and considered to be within the scope of the invention.

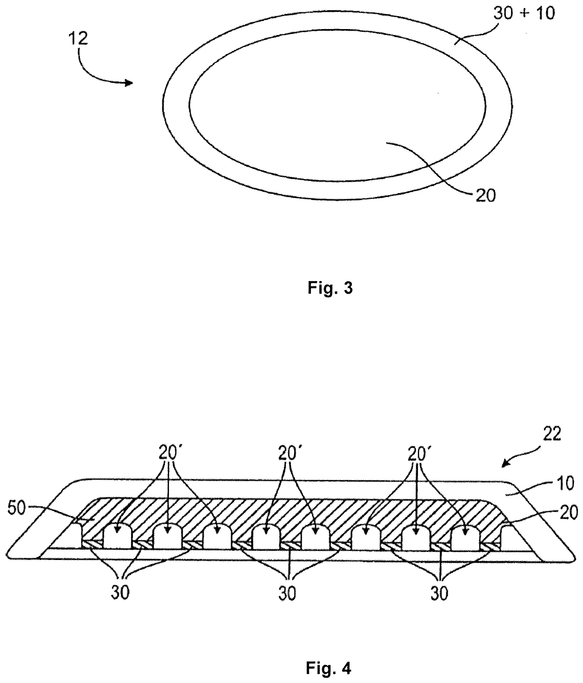

Referring to the embodiment shown in Figure 3, the textile composite material **12** has a carrier layer **10** having a planar surface area which is larger than a planar surface area of the active layer **20** so that the carrier layer **10** overlaps and encircles the rim of the active layer **20.** The overlapping edge of the carrier layer **10** is provided with an adhesive layer **30.** The adhesive layer **30** is provided on the surface of the composite material **12** that faces the skin to fasten the textile composite material **12** to the skin. This leads to an even better contact of the active layer **20** with the skin which in turn leads to an improvement of the decontaminating effect.

Furthermore, the adhesive layer **30** applied to the active layer **20** can be designed to remove the upper cell layer of the corneocytes from the skin. When removing the textile composite system **12** from the skin area to be decontaminated, the upper cell layer of the corneocytes, along with the noxious substance particles having already entered the latter, is thus extracted in a non-invasive way, and the proportion of the removed noxious substances is increased. In the embodiment shown, the shape of the textile composite material is chosen randomly and can of course be configured in any way.

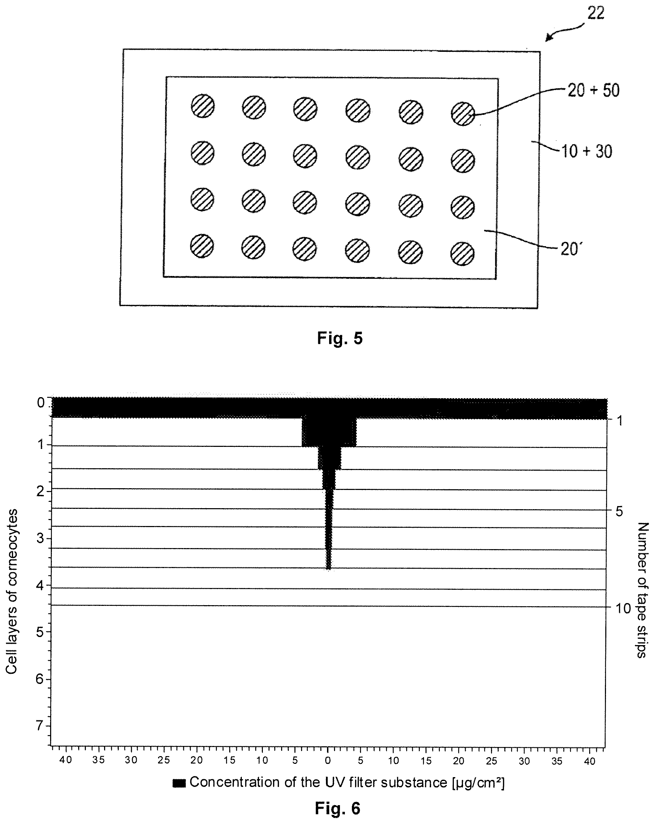

A further embodiment is shown with reference to Figs. 4 and 5. In this embodiment, the composite textile material **22** comprises the flexible carrier layer **10,** and a first and second active layers **20, 20',** wherein the first active layer **20** is provided with a metal coating **50.** The nanofiber nonwoven constituting the first active layer **20** is produced to form peaks and valleys. The valleys of the first active layer **20** are filled with the second active layer **20'.** At least the second active layer **20'** is comprised of the nanofiber nonwoven filled with superabsorbent. Preferably, both of the first and second active layers **20, 20'** include the superabsorbent. The adhesive layer **30** is provided on the metallic coating 50 (as shown in Fig. 4), or on the edge portion of the carrier layer **10** overlapping the rim of the active layers **20, 20'** (as shown in Fig. 5). The method for forming the peaks and valleys comprises manipulating the fibers during the spinning process either with a vacuum or with grids. The peaks and valleys were created to allow for both the absorptive component **20'** and the heat-conducting component **50** to be in direct contact with the skin. One benefit is that the metallic coating **50,** which acts as a heat-conducting component, needs to be in direct contact with the skin in order to be conductive. However, if the entire surface is coated, then the skin has no direct contact with the absorptive layer, and thus will not have optimal absorption of contaminate. By creating the peaks and valleys, both absorptive layer **20'** and the conductive layer **50** can be in direct contact with the skin.

The following is a description of the tests performed on volunteers to show the capability of the inventive textile composite material to effectively decontaminate the human skin.

### Test Materials and Methods

### A) Textile composite:

The absorbent textile composite used in these tests include an active layer comprised of a thermoplastic polyurethane-based nanofiber nonwoven filled with LUQUASORB™ (BASF SE, Ludwigshafen, Germany) having a skin-core structure and a particle size distribution of d50 = 55-100 µm and d100 = 100-150 µm. Other parameters of the nonwoven and composite were as follows:
a fiber diameter of between 300 nm and 1 µm;
an pore size of between 0.01 µm to 500 µm;
a porosity that is a percentage of the total volume of the nonwovens, which is free space, of about 80%;
a thickness of the active layer of about 0,5 mm; and
a mass per unit area of about 230g/m².

Further, the active layer comprised of the nanofiber nonwoven filled with the superabsorbent had the following properties:
a filling level of superabsorbent (calculated as weight by weight of dry material) of about 50%;
an absorbency (tb) measured in the teabag test in accordance with EDANA standard test WSP 240.2 (05) of about 28 g/g (saline, 0.9% NaCl in water, 30 min);
a retention capacity (CRC) measured in the centrifuge test according to EDANA standard test WSP 241.2 (05), of about 24 g/g; and
a contact angle as measured at 22 °C and 55% relative humidity (Fibro DAT™ of Rycobel, Belgium) of about 121°.

### B) Model formulation and skin treatment:

A waterproofed sunscreen containing 3% of the UV-filter substance octylmethoxycinnamate was applied onto the skin on the flexor forearm of 10 healthy volunteers. The sunscreen was chosen as a model formulation because it sticks strongly on the skin surface after application. 2 mg/cm² of the sunscreen was applied on selected skin areas at a size of 4 x 5 cm² for each area. The application areas were surrounded with a silicon barrier to avoid the spreading of the formulation on the skin surface. After 10 minutes penetration time, the penetration of the formulation into the skin was analyzed by the method of tape stripping as described below.

### C) Decontamination:

The decontamination was performed on one skin area by washing for 30 seconds under running water with soap.

A second skin area was used as a control without decontamination.

All other skin areas were decontaminated with the absorbent textile composite which was pressed onto the skin for 1 minute, without applying a washing or massage procedure, and finally removed.

### D) Tape Stripping:

The tape stripping test is based on the successive application and removal of adhesive films (Tesa Film, Beiersdorf, Hamburg, Germany) from the skin. The removed tape strips comprise approximately one cell layer of corneocytes and the corresponding part of topically applied substance localized within this cell layer. The amount of stratum corneum removed with a single tape strip is determined spectroscopically by determining the pseudo-absorption at 430 nm, whilst the concentration of the penetrated formulation is analyzed by the absorption of the UV-filter substance octylmethoxycinnamate at 310 nm.

Ten tape strips were removed from each skin area. The horny layer profile of the skin areas was calculated as described by Weigmann et. al., "Determination of the horny layer profile by tape stripping in combination with optical spectroscopy in the visible range as a prerequisite to quantify percutaneous absorption" in Skin Pharmacol. Appl. Skin Physiol., 1999, vol.12, pp. 34-45, which is incorporated by reference. Specifically, the horny layer profile of the skin areas was calculated by adding the pseudo-absorptions of the single tape strips removed from the same skin area. The penetration profile was determined by relating the amount of the penetrated UV filter substance to the corresponding tape strip in the horny layer profile. A typical example is shown in Fig. 6, wherein the distance between the horizontal lines corresponds to the amount of stratum corneum removed with a single tape strip. The upper horizontal lines represent the skin surface and the lower horizontal lines correspond to deeper parts of the stratum corneum.

The concentration of the UV filter octylmethoxycinnamate in the different samples was determined by absorption, using an UV/VIS spectrometer. The UVNIS spectra of the extracts were measured between 240 and 500 nm. The concentration of the UV filter substance was calculated from the determined absorption maximum at 310 nm on the basis of a calibration curve in ethanol.

### E) In vivo laser scanning microscopy (LSM):

The commercially available in vivo laser scanning microscope (Stratum™, Optilas, Melbourne, Australia) was used for the detection of the fluorescent model substance on the skin surface and in the upper layers of the skin. The excitation wavelength of the Argon laser used was 480 nm. The basic station of the LSM was connected to a handpiece with optical fibers. Optical imaging and focus systems were located within the handpiece. Skin areas under investigation were sized at 250 x 250 µm².

The fluorescence intensity and the distribution of the model substance was determined on all skin areas immediately before and after the decontamination.

### Results

Figure 6 is a graph illustrating the penetration profile of a model substance into the skin obtained by tape stripping. Specifically, Fig. 6 shows the typical penetration profile of the UV filter octylmethoxycinnamate, 10 minutes after application without decontamination obtained from skin area A. Most of the formulation is located in the first cell layers. The UV filter substances could be detected up to the 7^{th} cell layers of corneocytes. About 90% of the topically applied UV filter can be detected in the first 10 tape strips.

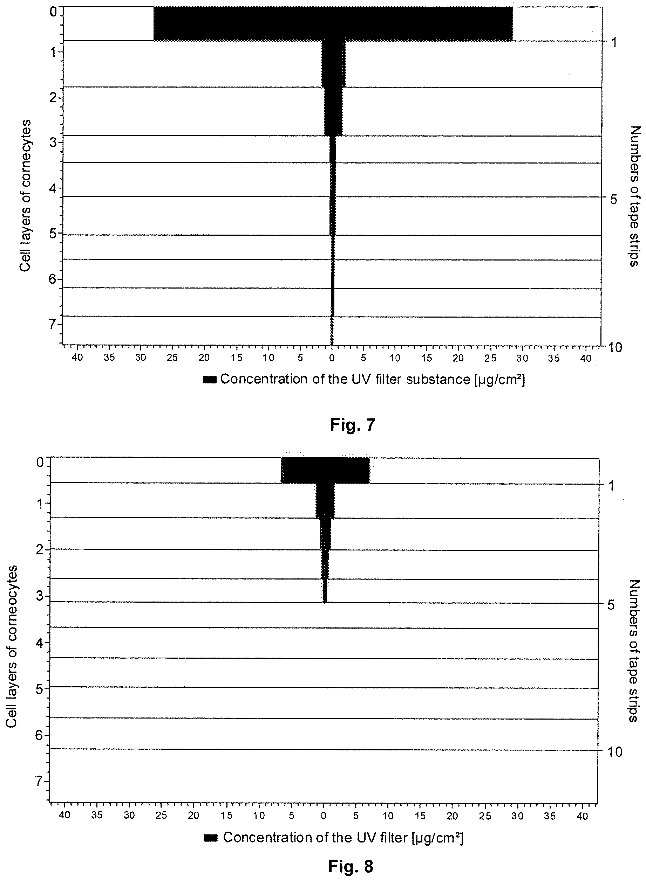

Figure 7 is a graph illustrating the distribution of the model substance in the stratum corneum after washing. Specifically, in Fig. 7, the distribution of the UV filter substance in the stratum corneum after washing is demonstrated. The results depicted in Fig. 7 show that the amount of UV filter substance in the upper cell layer was reduced by the washing procedure to about 60%. However, the UV filter substance could be detected in deeper layers in comparison to the penetration profile shown in Fig. 6.

Figure 8 is a graph illustrating the distribution of the model substance in the stratum corneum after decontamination with a textile composite material composed of nanofiber nonwoven filled with superabsorbent. Specifically, in Fig. 8, the penetration profiles after decontamination with the absorbent textile composite material are presented. In this case, the concentration of the octylmethoxycinnamate was reduced strongly in the stratum corneum in comparison to the washed skin area B. Specifically, a reduction to about 35% of the initial UV filter concentration was found in the case of skin decontamination with the textile composite material. A penetration in deeper parts of the stratum corneum as in the case of washing was not observed.

Similar results were obtained for use of an absorbent textile composite comprising an active layer comprised of the nanofiber nonwoven and the superabsorbent, and an absorbent textile composite material having a sandwich structure, respectively, wherein the active layer comprises a top and bottom cover layer formed from a nanofiber nonwoven without a superabsorbent, and a base layer comprised of the nanofiber nonwoven filled with superabsorbent and arranged between said top and bottom cover layer.

Therefore, use of the textile composite material for decontaminating the skin resulted in a removal of about 70% of the model formulation from the skin. In the case of decontamination with the textile composite, no massage was applied, so that the decontamination procedure does not stimulate the penetration into the hair follicles. Usually, hair follicles act as a long - term reservoir for topically applied substances providing significantly increased storage times in comparison to the stratum corneum. Additionally, the hair follicles contain or are surrounded by several important target structures, such as blood capillaries, stem and dendritic cells. Here, hazardous substances can exhibit strong destructive activities. The advantage of decontamination with the absorbent textile composite materials is that it can be carried out immediately without the utilization of water, which is not always available at the required time. Efficient methods for skin decontamination may be not only important in the case of industrial or research accidents, but also in relation to terrorist attacks.

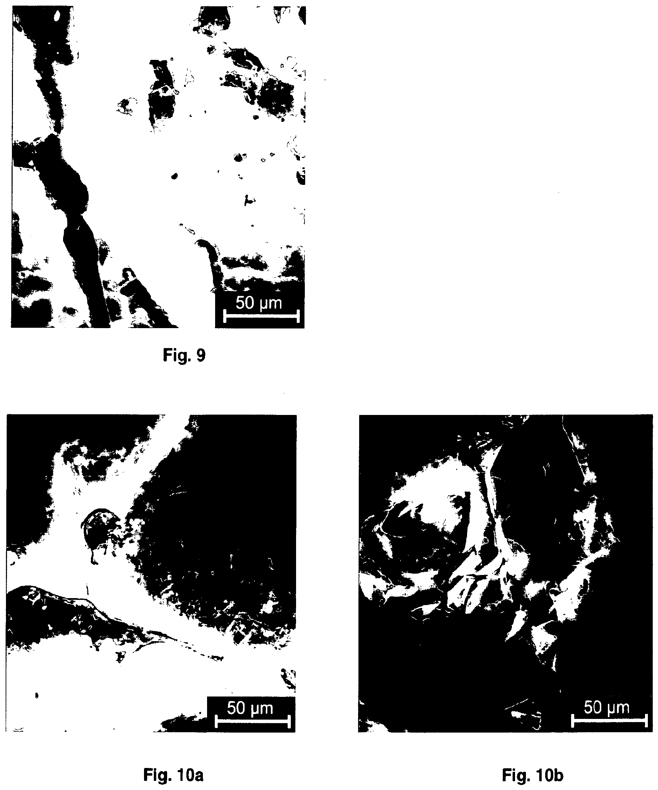

Typical images of the distribution of the fluorescent model substance on the skin surface by LSM measurements, with and without decontamination, are presented in Figs. 9 to 11. Specifically, Figure 9 illustrates an LSM image of the distribution of a fluorescent dye on the skin after application and penetration. Without decontamination, a strong fluorescent signal was detected on the skin surface (Fig. 9).

Figures 10a-b illustrate an LSM image of the distribution of the fluorescent dye after washing. The washing procedure led to a removal of the topically applied substance from the skin surface. However, a strong fluorescent signal was still localized in the region of the furrows and orifices of the hair follicles (Figs. 10a, b).

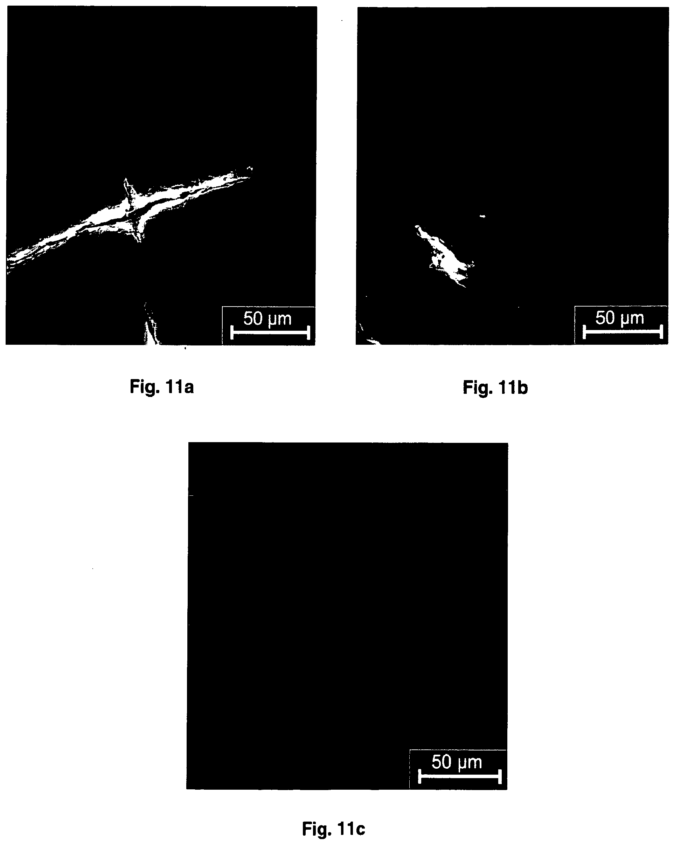

Figures 11a-c illustrate an LSM image of the distribution of the fluorescent dye after decontamination with the textile composite material. After decontamination with the absorbent material, the fluorescent signal was markedly reduced, both on the skin surface as well as in the furrows and orifices of the hair follicles. However, in Figs. 11a-b, a low fluorescent signal was still detectable in the area of the furrows and orifices of the hair follicles (Fig. 11a, b).

Furthermore, it was found that extending the application time of the, absorbent textile composite beyond 1 minute did not improve the decontamination effect. However, as shown in Fig. 11c, a repeated application of the absorbent material on the same skin area led to an almost complete removal of the fluorescent model substance from the furrows and orifices of the hair follicles (Fig. 11c).

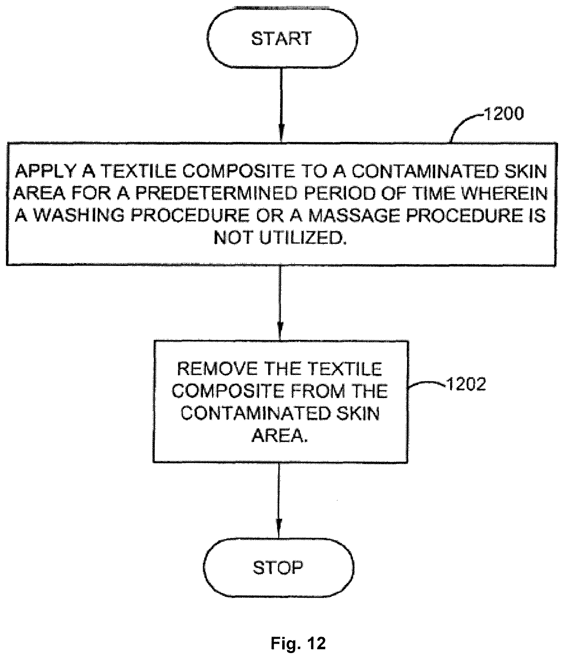

Figures 12-13 illustrate methodologies of decontaminating human skin from noxious substances. While, for purposes of simplicity of explanation, the one or more methodologies shown herein (*e.g*., in the form of a flow chart or flow diagram) are shown and described as a series of acts, it is to be understood and appreciated that the subject method is not limited by the order of acts, as some acts may, in accordance therewith, occur in a different order and/or concurrently with other acts from that shown and described herein. For example, those skilled in the art will understand and appreciate that a methodology could alternatively be represented as a series of interrelated states or events, such as in a state diagram. Moreover, not all illustrated acts may be required to implement the disclosed methodology.

Referring to Figure 12, a method of decontaminating human skin from noxious substances is illustrated. At **1200,** a textile composite is applied to a contaminated skin area for a predetermined period of time. The textile composite is applied to the contaminated skin area without applying a washing procedure or a massage procedure. Furthermore, the textile composite comprises an active layer comprising a nanofiber nonwoven which comprises a superabsorbent for absorbing and retaining at least one noxious substance. And, at **1202** the textile composite is removed from the contaminated skin area.

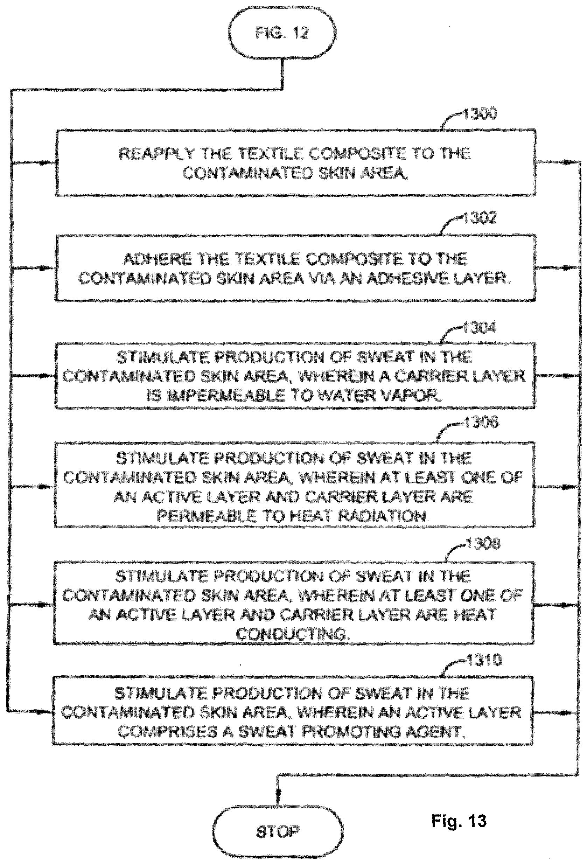

Referring to Figure 13, the method of decontaminating human skin from noxious substances is further illustrated. At **1300,** the textile composite is reapplied to the contaminated skin area. As stated *supra,* it was found that extending the application time of the textile composite beyond the predetermined time period did not improve the decontamination effect. However, a repeated application of the textile composite on the same contaminated skin area led to an almost complete removal of the noxious substance from the contaminated area as well as the furrows and orifices of the hair follicles.

At **1302,** the textile composite is adhered to the contaminated skin area via an adhesive layer. The adhesive layer is applied to the active layer and connects the composite material with the contaminated skin area. Furthermore, the adhesive layer can be designed to remove the upper cell layer of the corneocytes from the skin. When removing the textile composite from the contaminated skin area, the upper cell layer of the corneocytes, along with the noxious substance particles is extracted in a non-invasive way, and the proportion of the removed noxious substances is increased.

At **1304,** the production of sweat is stimulated in the contaminated skin area, wherein a carrier layer is impermeable to water vapor. An increased production of sweat by the parts of the skin covered by the active layer or carrier layer increases the decontaminating effect of the textile composite. The sweat flushes out the noxious substances having already penetrated the hair follicles and the upper cell layer of the corneocytes. The sweat, along with these noxious substances and the particles of noxious substances still present on the skin surface, is then absorbed by the superabsorbent in the nanofiber nonwoven and retained therein. In a specific embodiment, the textile composite material has a carrier layer that is impermeable to water vapor. The carrier layer which is impermeable to water vapor closes the active layer lying on the contaminated skin surface in a damp-tight manner and thus stimulates the production of sweat in the enclosed skin area.

At **1306,** the production of sweat is stimulated in the contaminated area, wherein at least one of the active layer and the carrier layer are configured to be permeable to heat radiation. At least one of the carrier layer and the active layer are designed to stimulate the production of sweat by the skin. The skin areas concerned which are covered by the textile composite can then be purposefully heated using heat radiators, for example, or a chemical reaction producing heat. A quick and controlled stimulation of the production of sweat in the contaminated skin area is thereby obtained.

At **1308,** the production of sweat is stimulated in the contaminated area, wherein at least one of an active layer and a carrier layer of the textile composite material are configured to be heat conducting. It is, for example, possible to insert heat conducting fibers or heat conducting filaments into the carrier layer and/or the active layer, or provide the active layer with a heat conducting coating. As in the embodiment described above, the skin areas concerned can be purposefully heated using appropriate heating sources such as, *e.g*., an electrical heating element, and the production of sweat can therefore be stimulated.

At **1310,** the production of sweat is stimulated in the contaminated area, wherein an active layer comprises an agent promoting the production of sweat. For example, by applying a sweat promoting agent onto the surface of the active layer that faces the skin, the agent will stimulate the production of sweat to flush out the noxious substances. The textile composite material then acts like a transdermal system, the agent being absorbed by the skin first and stimulating the production of sweat.

Suitable sweat producing agents may be methyl nicotinate, 2-hydroxyethyl salicylate, methyl salicylate, ethyl salicylate, menthol B.P. or agents containing benzene derivatives disclosed for example in JP-A 10114649.

Furthermore, the active layer may also comprise a color indicator to indicate the production of sweat. Owing to this addition, the optimum time to remove the material from the skin can be indicated to the user of the textile composite material. Color indicators appropriate for this use are for example: cobalt (II) chloride, quinizarin powder, pentamethoxy red, methyl yellow, phenolphthalein, thymolphthalein, p-naphtolbenzein, 4-nitrophenol, 3-nitrophenol, o-cresolphthalein, m-cresol red, thymol blue, m-cresol purple, or mixtures thereof, the colors of which change if the nanofiber nonwoven has absorbed an amount of sweat that is sufficient for decontaminating and/or the capacity of the superabsorbent to absorb liquid has been exhausted. The color indicator may be combined with the superabsorbent and incorporated into the active layer, for example by adding the combined superabsorbent and color indicator to the polymer melt or solution before spinning of the nanofiber nonwoven.

The embodiments described above can be combined with each other in any way. The carrier layer can be at the same time impermeable to water vapor and heat conducting or permeable to heat radiation, and/or the active layer can contain an agent promoting the production of sweat. Further combinations are also conceivable and considered to be within the scope of the invention.

The carrier layer and the active layer can be formed integrally with each other. For example, the carrier layer can be a woven fabric, and the nanofibers of the active layer can be firmly spun onto and with the woven fabric filaments of the carrier layer. Preferably, the carrier layer and the active layer are bonded to each other. The layers can then be produced separately, so as to have the respective desired properties, and then bonded to each other by chemical, thermal or physical bonding as is generally known in the art.

Additionally, it is also contemplated that the above layers can be interchangeable without affecting the overall concept of the invention. Illustrative aspects are described herein in connection with the following description and the annexed drawings to further illustrate such interchangeability. These aspects are indicative, however, of but a few of the various ways in which the layers disclosed herein can be employed and interchanged, and the contemplated invention is intended to include all such aspects and their equivalents.

Further, what has been described above includes examples of the claimed subject matter. It is, of course, not possible to describe every conceivable combination of components or methodologies for purposes of describing the claimed subject matter, but one of ordinary skill in the art may recognize that many further combinations and permutations of the claimed subject matter are possible. Accordingly, the claimed subject matter is intended to embrace all such alterations, modifications and variations that fall within the scope of the appended claims. Furthermore, to the extent that the term "includes" or "include" is used in either the detailed description or the claims, such term is intended to be inclusive in a manner similar to the term "comprising" as "comprising" is interpreted when employed as a transitional word in a claim.

## Claims

1. An absorbent textile composite material comprising a carrier layer and an active layer, wherein the active layer is connected with the carrier layer and comprises a nanofiber nonwoven optionally filled with a superabsorbent
the carrier layer comprises cellulose, derivatives of cellulose, polyglycolide, polylactic acid, polyethylene adipate, polyhydroxyalkanoate, polybutylene terephthalate, polytrimethylene terephthalate, polyethylene naphthalate, or a mixture or copolymer thereof;
the carrier layer is impermeable to water vapor; and
the nanofiber nonwoven comprises fibers with a diameter of between 0.001 µm and 10 µm.

2. The absorbent textile composite material of claim 1, wherein the carrier layer and the active layer are integrally formed with or bonded to each other.

3. The absorbent textile composite material of claim 1, wherein the carrier layer is flexible, elastic, not expandable, permeable to heat radiation, or heat-conducting.

4. The absorbent textile composite material of claim 1, wherein the active layer further comprises a heat conductive coating, a color indicator to indicate production of sweat, or an agent that is capable of stimulating production of sweat on a skin or reacting to neutralize noxious substances.

5. The absorbent textile composite material of claim 1, wherein the optional superabsorbent comprises a starch graft polymer, a biodegradable superabsorbent, activated carbon, clay, aluminum oxide, ionic exchange resin, polyacrylate, or polymer particles which have a core swelling in the presence of water and a superficially postcured shell.

6. The absorbent textile composite material of claim 5, wherein the polymer particles having a core swelling in the presence of water are a screening fraction of polymer particles which have not been crushed after superficial postcure of the shell.

7. The absorbent textile composite material of claim 1, wherein the nanofiber nonwoven comprises cellulose, cellulose derivative, polyurethane, polyamide, polyester, polyacrylonitrile, polyvinyl alcohol, polyvinyl pyrrolidone, polyethylene oxide, cellulose acetate, poly(ethylenimine), poly(caprolactone), poly(2-hydroxymethacrylate), or a mixture or copolymer thereof.

8. The absorbent textile composite material of claim 1, wherein the carrier layer is larger than the active layer and comprises an adhesive layer encircling its rim to connect the composite material with the skin.

9. The absorbent textile composite material of claim 1, further comprising at least one layer of a nanofiber nonwoven without the superabsorbent, or an adhesive layer optionally positioned on the active layer.

10. The absorbent textile composite material of claim 1, wherein the nanofiber nonwoven comprises fibers with a diameter of between 0.1 µm and 1.5 µm, or between 300 nm and 900 nm.

11. The absorbent textile composite material of claim 1, wherein the nanofiber nonwoven has an average pore size of between 0.01 µm and 500 µm, less than 250 µm, or less than 100 µm.

12. The absorbent textile composite material of claim 1, wherein the nanofiber nonwoven has a porosity of between 10% and 90%, between 40% and 90%, or between 70% and 90%.

13. The absorbent textile composite material of claim 1, wherein the nanofiber nonwoven has a density of between 0.5 g/cm³ and 1.5 g/cm³ or between 0.8 g/cm³ and 1.5 g/cm³.

14. The absorbent textile composite material of claim 1, wherein the nanofiber nonwoven has a mass per unit area of between 5 g/m² and 1000 g/m², between 50 g/m² and 500 g/m², between 50 g/m² and 400 g/m², or between 150 g/m² and 250 g/m².

15. The absorbent textile composite material of claim 1, wherein the nanofiber nonwoven has a breaking force of 0.1 to 100 MPa or 0.5 to 5.0 MPa, and elongation of 100% to 2,000% or 250% to 1,000%

16. The absorbent textile composite material of claim 17, wherein the nanofiber nonwoven has a breaking force of 1.5 to 2.0 MPa and elongation of 400% to 500%.

17. The absorbent textile composite material of claim 1, wherein the nanofiber nonwoven has an absorbency in saline of between about 0 g/g and about 200 g/g, between about 2.5 g/g and about 150.0 g/g, or between about 8.0 g/g and about 10.0 g/g.

18. The absorbent textile composite material of claim 1, wherein the nanofiber nonwoven has a retention capacity for saline of between about 0 g/g and about 200 g/g, between about 3.0 g/g and about 25.0 g/g, or between about 6.0 g/g and about 8.0 g/g.

19. The absorbent textile composite material of claim 1, wherein the active layer has a filling level of superabsorbent of between about 0.1% and about 80%, between about 10% and about 80%, between about 40% and about 80%, or between about 50% and about 75%.

20. The absorbent textile composite material of claim 1, wherein the active layer has an absorbency in saline of between about 10 g/g and about 100 g/g at a superabsorbent filling level of 50%, between about 20 g/g and about 75 g/g at a superabsorbent filling level of 75%, at least 0.01 g/g without superabsorbent, between about 25 g/g and about 31 g/g at a superabsorbent filling level of 50%, or between about 38 g/g and about 45 g/g at a superabsorbent filling level of 75%.

21. The absorbent textile composite material of claim 1, wherein the active layer has a retention capacity for saline of between 14 g/g and 40 g/g at a superabsorbent filling level of between 50% and 75%, or between 20 g/g and 35 g/g at a superabsorbent filling level of between 50% and 75%.

22. Use of an absorbent textile composite material of any of claims 1 to 21 for the manufacture of a kit for decontaminating a skin area contaminated with noxious substances.

## Patentansprüche

1. Absorptionsfähiger, textiler Verbundstoff mit einer Trägerschicht und einer aktiven Schicht, wobei die aktive Schicht mit der Trägerschicht verbunden ist und ein Nanofaservlies umfasst, das optional mit einem Superabsorber gefüllt ist,
wobei die Trägerschicht Cellulose, Derivate von Cellulose, Polyglycolid, Polylactid, Polyethylenadipat, Polyhydroxyalkanoat, Polybutylenterephthalat, Polytrimethylenterephthalat, Polyethylennaphthalat oder eine Mischung oder ein Copolymer davon umfasst,
die Trägerschicht wasserdampfundurchlässig ist und
das Nanofaservlies Fasern mit einem Durchmesser zwischen 0,001 µm und 10 µm umfasst.

2. Absorptionsfähiger, textiler Verbundstoff nach Anspruch 1, bei dem die Trägerschicht und die aktive Schicht einstückig miteinander ausgebildet oder haftend miteinander verbunden sind.

3. Absorptionsfähiger, textiler Verbundstoff nach Anspruch 1, bei dem die Trägerschicht flexibel, elastisch, nicht dehnbar, für Wärmestrahlung durchlässig oder wärmeleitend ist.

4. Absorptionsfähiger, textiler Verbundstoff nach Anspruch 1, bei dem die aktive Schicht ferner eine wärmeleitende Beschichtung, einen Farbindikator zur Anzeige einer Schweißbildung oder einen Wirkstoff umfasst, der die Schweißbildung auf einer Haut anregen oder so reagieren kann, dass Schadstoffe neutralisiert werden.

5. Absorptionsfähiger, textiler Verbundstoff nach Anspruch 1, bei dem der optionale Superabsorber ein Stärke-Pfropfpolymer, einen biologisch abbaubaren Superabsorber, Aktivkohle, Ton, Aluminiumoxid, Ionenaustauschharz, Polyacrylat oder Polymerpartikel umfasst, die einen in Gegenwart von Wasser aufquellenden Kern und eine oberflächlich nachvernetzte Schale aufweisen.

6. Absorptionsfähiger, textiler Verbundstoff nach Anspruch 5, bei dem die Polymerpartikel, die einen in Gegenwart von Wasser aufquellenden Kern aufweisen, eine Siebfraktion solcher Polymerpartikel sind, die nach der oberflächlichen Nachvernetzung der Schale nicht zerkleinert worden sind.

7. Absorptionsfähiger, textiler Verbundstoff nach Anspruch 1, bei dem das Nanofaservlies Cellulose, Cellulosederivat, Polyurethan, Polyamid, Polyester, Polyacrylnitril, Polyvinylalkohol, Polyvinylpyrrolidon, Polyethylenoxid, Celluloseacetat, Poly(ethylenimin), Poly(caprolacton), Poly(2-hydroxymethacrylat) oder eine Mischung oder ein Copolymer davon umfasst.

8. Absorptionsfähiger, textiler Verbundstoff nach Anspruch 1, bei dem die Trägerschicht größer als die aktive Schicht ist und eine um ihren Rand umlaufende Haftschicht umfasst, um den Verbundstoff mit der Haut zu verbinden.

9. Absorptionsfähiger, textiler Verbundstoff nach Anspruch 1, ferner mit wenigstens einer Schicht eines Nanofaservlieses ohne den Superabsorber oder einer Haftschicht, die optional auf der aktiven Schicht angeordnet ist.

10. Absorptionsfähiger, textiler Verbundstoff nach Anspruch 1, bei dem das Nanofaservlies Fasern mit einem Durchmesser zwischen 0,1 µm und 1,5 µm oder zwischen 300 nm und 900 nm umfasst.

11. Absorptionsfähiger, textiler Verbundstoff nach Anspruch 1, bei dem das Nanofaservlies eine mittlere Porengröße zwischen 0,01 µm und 500 µm, kleiner als 250 µm oder kleiner als 100 µm hat.

12. Absorptionsfähiger, textiler Verbundstoff nach Anspruch 1, bei dem das Nanofaservlies eine Porosität zwischen 10% und 90%, zwischen 40% und 90% oder zwischen 70% und 90% hat.

13. Absorptionsfähiger, textiler Verbundstoff nach Anspruch 1, bei dem das Nanofaservlies eine Dichte zwischen 0,5 g/cm³ und 1,5 g/cm³ oder zwischen 0,8 g/cm³ und 1,5 g/cm³ hat.

14. Absorptionsfähiger, textiler Verbundstoff nach Anspruch 1, bei dem das Nanofaservlies eine flächenbezogene Masse zwischen 5 g/m² und 1.000 g/m², zwischen 50 g/m² und 500 g/m², zwischen 50 g/m² und 400 g/m² oder zwischen 150 g/m² und 250 g/m² hat.

15. Absorptionsfähiger, textiler Verbundstoff nach Anspruch 1, bei dem das Nanofaservlies eine Höchstzugkraft von 0,1 bis 100 MPa oder 0,5 bis 5,0 MPa und eine Bruchdehnung von 100% bis 2.000% oder 250% bis 1.000% hat.

16. Absorptionsfähiger, textiler Verbundstoff nach Anspruch 17, bei dem das Nanofaservlies eine Höchstzugkraft von 1,5 bis 2,0 MPa und eine Bruchdehnung von 400% bis 500% hat.

17. Absorptionsfähiger, textiler Verbundstoff nach Anspruch 1, bei dem das Nanofaservlies ein Absorptionsvermögen in Kochsalzlösung zwischen etwa 0 g/g und etwa 200 g/g, zwischen etwa 2,5 g/g und etwa 150,0 g/g oder zwischen etwa 8,0 g/g und etwa 10,0 g/g hat.

18. Absorptionsfähiger, textiler Verbundstoff nach Anspruch 1, bei dem das Nanofaservlies ein Vermögen zur Retention von Kochsalzlösung zwischen etwa 0 g/g und etwa 200 g/g, zwischen etwa 3,0 g/g und etwa 25,0 g/g oder zwischen etwa 6,0 g/g und etwa 8,0 g/g hat.

19. Absorptionsfähiger, textiler Verbundstoff nach Anspruch 1, bei dem die aktive Schicht einen Füllgrad an Superabsorber zwischen etwa 0,1% und etwa 80%, zwischen etwa 10% und etwa 80%, zwischen etwa 40% und etwa 80% oder zwischen etwa 50% und etwa 75% hat.

20. Absorptionsfähiger, textiler Verbundstoff nach Anspruch 1, bei dem die aktive Schicht ein Absorptionsvermögen in Kochsalzlösung zwischen etwa 10 g/g und etwa 100 g/g bei einem Superabsorberfüllgrad von 50%, zwischen etwa 20 g/g und etwa 75 g/g bei einem Superabsorberfüllgrad von 75%, von mindestens 0,01 g/g ohne Superabsorber, zwischen etwa 25 g/g und etwa 31 g/g bei einem Superabsorberfüllgrad von 50% oder zwischen etwa 38 g/g und etwa 45 g/g bei einem Superabsorberfüllgrad von 75% hat.

21. Absorptionsfähiger, textiler Verbundstoff nach Anspruch 1, bei dem die aktive Schicht ein Vermögen zur Retention von Kochsalzlösung zwischen 14 g/g und 40 g/g bei einem Superabsorberfüllgrad zwischen 50% und 75% oder zwischen 20 g/g und 35 g/g bei einem Superabsorberfüllgrad zwischen 50% und 75% hat.

22. Verwendung eines absorptionsfähigen, textilen Verbundstoffs nach einem der Ansprüche 1 bis 21 zur Herstellung eines Ausrüstungssatzes zur Dekontaminierung eines mit Schadstoffen kontaminierten Hautbereichs.

## Revendications

1. Matériau composite textile absorbant, comportant une couche support et une couche active, la couche active étant reliée à la couche support et comprenant un non-tissé de nanofibres rempli en option d'un superabsorbant,
la couche support comprenant de la cellulose, des dérivatifs de cellulose, du polyglycolide, de l'acide polylactique, de l'adipate de polyéthylène, du polyhydroxyalcanoate, du polytéréphtalate de butylène, du polytéréphtalate de triméthylène, du polynaphtalate d'éthylène ou un mélange ou un copolymère de ceux-ci;
la couche support étant imperméable à la vapeur d'eau; et
le non-tissé de nanofibres comprenant des fibres d'un diamètre compris entre 0,001 µm et 10 µm.

2. Matériau composite textile absorbant selon la revendication 1, la couche support et la couche active étant réalisées d'une seule pièce ou étant reliées l'une à l'autre par adhérence.

3. Matériau composite textile absorbant selon la revendication 1, la couche support étant flexible, élastique, non extensible, perméable au rayonnement thermique ou conductrice de la chaleur.

4. Matériau composite textile absorbant selon la revendication 1, la couche active comprenant en outre un revêtement conducteur de chaleur, un indicateur coloré pour indiquer la production de sueur ou un agent apte à stimuler la production de sueur sur la peau ou réagissant de manière à neutraliser les substances nocives.

5. Matériau composite textile absorbant selon la revendication 1, le superabsorbant optionnel comprenant un polymère greffé d'amidon, un superabsorbant biodégradable, du charbon actif, de l'argile, de l'oxyde d'aluminium, de la résine échangeuse d'ions, du polyacrylate ou des particules polymères présentant un noyau gonflant en présence d'eau et une enveloppe post-réticulée superficiellement.

6. Matériau composite textile absorbant selon la revendication 5, les particules polymères qui présentent un noyau gonflant en présence d'eau étant une fraction de tamisage du type de particules polymères qui n'ont pas été broyées après la post-réticulation superficielle de l'enveloppe.

7. Matériau composite textile absorbant selon la revendication 1, le non-tissé de nanofibres comprenant de la cellulose, un dérivatif de cellulose, du polyuréthane, du polyamide, du polyester, du polyacrylonitrile, de l'alcool polyvinylique, de la polyvinylpyrrolidone, de l'oxyde de polyéthylène, de l'acétate de cellulose, de la poly(éthylènimine), de la poly(caprolactone), du poly(2-hydroxyméthacrylate) ou un mélange ou un polymère de ceux-ci.

8. Matériau composite textile absorbant selon la revendication 1, la couche support étant plus grande que la couche active et présentant une couche adhésive entourant son bord pour relier le matériau composite à la peau.

9. Matériau composite textile absorbant selon la revendication 1, comprenant en outre au moins une couche d'un non-tissé de nanofibres sans ledit superabsorbant ou une couche adhésive agencée en option sur la couche active.

10. Matériau composite textile absorbant selon la revendication 1, le non-tissé de nanofibres comportant des fibres d'un diamètre compris entre 0,1 µm et 1,5 µm ou entre 300 nm et 900 nm.

11. Matériau composite textile absorbant selon la revendication 1, le non-tissé de nanofibres présentant une taille de pore moyenne comprise entre 0,01 µm et 500 µm, inférieure à 250 µm ou inférieure à 100 µm

12. Matériau composite textile absorbant selon la revendication 1, le non-tissé de nanofibres présentant une porosité comprise entre 10 % et 90 %, entre 40 % et 90 % ou entre 70 % et 90 %.

13. Matériau composite textile absorbant selon la revendication 1, le non-tissé de nanofibres présentant une densité comprise entre 0,5 g/cm³ et 1,5 g/cm³ ou entre 0,8 g/cm³ et 1,5 g/cm³.

14. Matériau composite textile absorbant selon la revendication 1, le non-tissé de nanofibres présentant une masse surfacique comprise entre 5 g/m² et 1000 g/m², entre 50 g/m² et 500 g/m², entre 50 g/m² et 400 g/m², ou entre 150 g/m² et 250 g/m².

15. Matériau composite textile absorbant selon la revendication 1, le non-tissé de nanofibres présentant une force de rupture de 0,1 à 100 MPa ou de 0,5 à 5,0 MPa, et un allongement de 100 % à 2000 % ou de 250 % à 1000 %.

16. Matériau composite textile absorbant selon la revendication 17, le non-tissé de nanofibres présentant une force de rupture de 1,5 à 2,0 MPa et un allongement de 400 % à 500 %

17. Matériau composite textile absorbant selon la revendication 1, le non-tissé de nanofibres présentant une capacité d'absorption saline comprise entre environ 0 g/g et environ 200 g/g, entre environ 2,5 g/g et environ 150,0 g/g, ou entre environ 8,0 g/g et environ 10,0 g/g.

18. Matériau composite textile absorbant selon la revendication 1, le non-tissé de nanofibres présentant une capacité de rétention saline comprise entre environ 0 g/g et environ 200 g/g, entre environ 3,0 g/g et environ 25,0 g/g, ou entre environ 6,0 g/g et environ 8,0 g/g.

19. Matériau composite textile absorbant selon la revendication 1, la couche active présentant un niveau de remplissage en superabsorbant compris entre environ 0,1 % et environ 80 %, entre environ 10 % et environ 80 %, entre environ 40 % et environ 80%, ou entre environ 50 % et environ 75 %.

20. Matériau composite textile absorbant selon la revendication 1, la couche active présentant une capacité d'absorption saline comprise entre environ 10 g/g et environ 100 g/g à un niveau de remplissage de superabsorbant de 50 %, entre environ 20 g/g et environ 75 g/g à un niveau de remplissage de superabsorbant de 75 %, au moins 0,01 g/g sans superabsorbant, entre environ 25 g/g et environ 31 g/g à un niveau de remplissage de superabsorbant de 50 %, ou entre environ 38 g/g et environ 45 g/g à un niveau de remplissage de superabsorbant de 75 %.

21. Matériau composite textile absorbant selon la revendication 1, la couche active présentant une capacité de rétention saline comprise entre 14 g/g et 40 g/g à un niveau de remplissage de superabsorbant entre 50 % et 75 % ou entre 20 g/g et 35 g/g à un niveau de remplissage de superabsorbant entre 50 % et 75 %.

22. Utilisation d'un matériau composite textile absorbant selon l'une des revendications 1 à 21 pour la production d'un jeu pour la décontamination d'une zone de la peau contaminée de substances nocives.
